**Europäisches Patentamt**

**European Patent Office**

**Office européen des brevets**

(11) Veröffentlichungsnummer: **0 038 908**
**B1**

(12) **EUROPÄISCHE PATENTSCHRIFT**

(45) Veröffentlichungstag der Patentschrift:
**14.08.85**

(21) Anmeldenummer: **81100935.6**

(22) Anmeldetag: **11.02.81**

(51) Int. Cl.⁴: **A 61 F 2/36**

(54) **Hüftgelenkprothese mit einem in den Markkanal des Oberschenkelknochens einzusetzenden Schaft.**

(30) Priorität: **24.04.80 DE 3015690**

(43) Veröffentlichungstag der Anmeldung:
**04.11.81 Patentblatt 81/44**

(45) Bekanntmachung des Hinweises auf die Patenterteilung:
**14.08.85 Patentblatt 85/33**

(84) Benannte Vertragsstaaten:
**CH FR GB IT LI SE**

(56) Entgegenhaltungen:
**DE - A - 2 063 547**
**DE - A - 2 851 598**
**DE - B - 2 247 560**
**DE - U - 8 012 922**
**GB - A - 1 030 145**
**GB - A - 1 126 961**
**US - A - 3 510 883**

(73) Patentinhaber: **Waldemar Link GmbH & Co,**
**Barkhausenweg 10, D-2000 Hamburg 63 (DE)**

(72) Erfinder: **Keller, Arnold, An der Naher Furth 5,**
**D-2061 Kaihude (DE)**

(74) Vertreter: **Glawe, Delfs, Moll & Partner Patentanwälte,**
**Rothenbaumchaussee 58 Postfach 2570,**
**D-2000 Hamburg 13 (DE)**

## Beschreibung

Die Erfindung bezieht sich auf eine Hüftgelenkprothese mit einem in den Markkanal des Oberschenkelknochens einzusetzenden, in der AP-Ebene gekrümmten Schaft mit posterior gelegenem Krümmungsmittelpunkt. Die AP-Ebene ist in diesem Zusammenhang die von anterior nach posterior in Knochen- bzw. Schaftrichtung verlaufende Ebene, die senkrecht steht auf der LM-Ebene (lateral-medial), die parallel zum Ober-schenkelknochen verläuft und dessen Kniekondy-len tangiert.

Es ist eine Hüftgelenkprothese der eingangs genannten Art bekannt, deren Schaft in der AP-Ebene eine Krümmung mit posterior gelegenem Krümmungsmittelpunkt besitzt. Dadurch wird eine Anpassung an den gekrümmten Verlauf des Markkanals und die Möglichkeit erreicht, den Schaft ohne grössere Schwächung des Knochens dicker und somit stärker auszuführen. Bei diesem bekannten Schaft legt man Wert auf eine gleichförmige Krümmung über seine ganze Länge, damit er leicht in die Knochenhöhlung eingesetzt werden kann.

Jedoch hat sich gezeigt, dass eine solche Schaftform eine stärkere Ausfräsung der Spongiosa im Bereich des kleinen Trochanter voraussetzt, die unerwünscht ist. Auch zeigt der Schaft mitunter eine Neigung, sich vor dem Erhärten des Zements zu verdrehen, weil der Markkanal nicht nur in der AP-Ebene, sondern auch in der LM-Ebene gekrümmt ist und daher je nach der individuellen Ausprägung des Markkanals die Möglichkeit besteht, dass eine von der vorgesehenen Einstellung des Schafts im Winkel abweichende Stellung eine bessere Formübereinstimmung verspricht. Bei den in der AP-Ebene gerade ausgebildeten, von der Praxis bislang bevorzugten Schäften ist diese Neigung weniger stark ausgeprägt. Im Vergleich damit verlangt der gekrümmte Schaft daher grössere Aufmerksamkeit während der Operation bezüglich seiner Winkelstellung.

Ferner ist eine Hüftgelenkprothese der eingangs genannten Art bekannt (GB-A-1 126 961), deren Schaft ausser der Krümmung in der LM-Ebene auch einen Knick in der AP-Ebene aufweist. Aus dem Vergleich der Schaftform mit der Gestalt des Oberschenkelknochens, der eine ausgeprägte Krümmung mit posterior gelegenem Krümmungsmittelpunkt aufweist, sowie aus dem Vergleich der in der Zeichnung mit durchgezogenen Linien dargestellten Form mit der in der Beschreibung an erster Stelle erläuterten Alternative kann man entnehmen, dass der zu dem Knick gehörige Krümmungsmittelpunkt des Prothesenschafts posterior liegt. Daraus ist zu schliessen, dass diese bekannte Prothese dieselben Nachteile besitzt wie der zuvor erläuterte Stand der Technik.

Der Erfindung liegt daher die Aufgabe zugrunde, eine Hüftgelenkprothese der eingangs genannten Art zu schaffen, die eine gute Anpassung an die natürlichen Gegebenheiten und damit eine beträchtliche Schaftdicke ermöglicht, die eine geringere Verdrehungsneigung hat und damit problemloser bezüglich der Operationstechnik ist und die schliesslich eine günstige Querschnitts- und Kraftverteilung im Trochanterbereich ermöglicht.

Die erfindungsgemässe Lösung besteht darin, dass der Schaft im proximalen Bereich eine Krüm-mung mit anterior gelegenem Krümmungsmittel-punkt aufweist, während die Krümmung mit posterior gelegenem Krümmungsmittelpunkt sich im distalen Schaftbereich befindet. Vorzugsweise ist vorgesehen, dass die Krümmung mit anterior gelegenem Krümmungsmittelpunkt innerhalb einer Strecke von etwa 4—8 cm unterhalb einer den Schaft proximal abschliessenden Pfanne liegt. Die durch die Krümmung bewirkte Richtungsänderung liegt zweckmässigerweise im Bereich von etwa 5—10°. Eine etwaige Krümmung des Schafts im LM-Bereich bleibt von der Erfindung unberührt.

Es hat sich gezeigt, dass der erfindungsgemässe Schaft im allgemeinen keine wesentliche Verdrehungsneigung im Verhältnis zur seiner vorgesehenen Winkelstellung zeigt. Dadurch wird die Operationstechnik vereinfacht. Die Anpassung an die natürlichen Gegebenheiten sind sehr gut. Ins-besondere führt die erfindungsgemässe Krüm-mung zu einer günstigen Lage des Schafts im Trochanterbereich. Das Knochengewebe im Bereich des kleinen Trochanters wird geschont, ohne dass sich auf der gegenüberliegenden Seite die zur Aufnahme des Schafts ausgearbeitete Höhlung zu sehr der Corticalis nähert oder diese gar schwächt. Es wird eine ausgezeichnete Krafteinleitung in den Trochanterbereich des Knochens erreicht, weil sich die Richtung des Schafts an dessen proximalem Ende derjenigen des Kopfhalses nähert und vorzugsweise (von lateral quer zum Kopfhals gesehen) damit sogar etwa übereinstimmt. Befürchtungen, dass sich der Schaft wegen seiner wechselnden Krümmung nicht oder nur mit Beschädigungen in den Markkanal einführen liesse, haben sich nicht bestätigt.

Die Erfindung wird im folgenden näher unter Bezugnahme auf die Zeichnung erläutert, die ein vorteilhaftes Ausführungsbeispiel veranschaulicht. Darin zeigen:

Fig. 1 eine Projektion einer linken Prothese auf die AP-Ebene von medial gesehen und

Fig. 2 eine entsprechende Projektion auf die LM-Ebene von dorsal.

Die Prothese besteht aus dem Schaft 1, der proximal durch die Pfanne 2 abgeschlossen wird, dem Kopfhals 3 und dem Kopf 4. In der Projektion auf die LM-Ebene ist der Schaft in einer Richtung gekrümmt, wobei die Zunahme der Schaftquerab-messung von distal nach proximal die Krümmungszunahme derart ausgleicht, dass der Schaft leicht in die Markhöhle des Knochens eingeführt werden kann, dessen Schnitt in beiden Darstellun-gen strichpunktiert angedeutet ist.

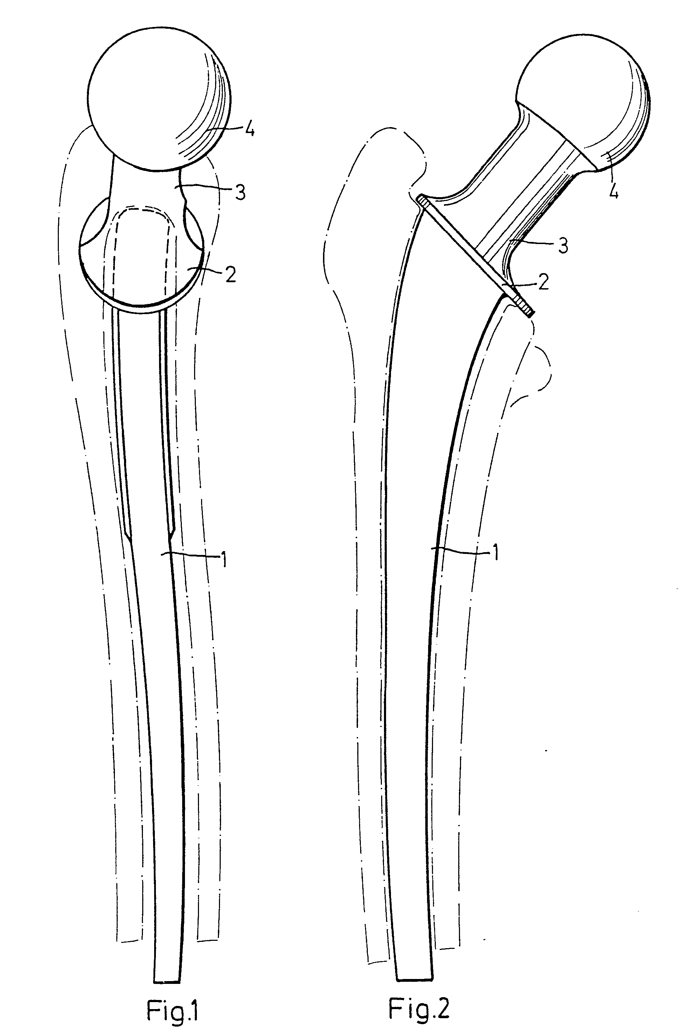

Die erfindungsgemässen Eigenschaften erge-ben sich aus Fig. 1, aus der man erkennt, dass de Schaft eine S-förmige Krümmung aufweist. In proximalen Bereich ist er auf der Vorderseite kon-kav, im distalen Bereich konvex. Die Querschnitts-abnahme nach unten hin ist verhältnismässig ge ring. Man erkennt, dass die Biegung des Schafts dem Verlauf der Markhöhle im mittleren und distalen Bereich sehr gut angepasst ist. Ferner erkennt man, dass im proximalen Bereich ebenfalls eine gute Anpassung erreicht ist durch entsprechenden Verlauf der in der Spongiosa gebildeten Höhlung, wobei auf der dorsalen Seite des Trochanterbereichs (links) mehr Knochenmaterial verbleibt als auf der ventralen (rechts), wenngleich die individuellen Bedingungen und der Operationsverlauf in dieser Hinsicht Abweichungen hervorrufen mögen. Der Bereich des kleinen Trochanters wird dadurch geschont.

Man erkennt in Fig. 1 die Anteversion des Kopfhalses, die durch eine gewisse Schrägung der Kopfhalsrichtung gegenüber der Gesamtrichtung der Anordnung nach rechts zum Ausdruck kommt. Man erkennt ferner, dass die erfindungsgemässe Krümmung des proximalen Schaftteils eine gewisse Angleichung an diese Richtung bringt. Dies spielt eine Rolle für einen harmonischen Spannungsverlauf in der Prothese und eine gute Krafteinleitung in den Knochen, insbesondere in dessen proximales Ende.

## Patentansprüche

1. Hüftgelenkprothese mit einem in den Markkanal des Oberschenkelknochens einzusetzenden, in der AP-Ebene gekrümmten Schaft mit posterior gelegenem Krümmungsmittelpunkt, dadurch gekennzeichnet, dass der Schaft (1) im proximalen Bereich eine Krümmung mit anterior gelegenem Krümmungsmittelpunkt aufweist, während die Krümmung mit posterior gelegenem Krümmungsmittelpunkt sich im distalen Schaftbereich befindet.

2. Prothese nach Anspruch 1, dadurch gekennzeichnet, dass die Krümmung mit anterior gelegenem Krümmungsmittelpunkt innerhalb einer Strecke von etwa 4–8 cm unterhalb einer den Schaft proximal abschliessenden Pfanne liegt.

3. Prothese nach Anspruch 1 oder 2, dadurch gekennzeichnet, dass die Krümmung mit anterior gelegenem Krümmungsmittelpunkt eine Richtungsänderung in der AP-Ebene von etwa 5–10° umfasst.

4. Prothese nach einem der Ansprüche 1 bis 4, dadurch gekennzeichnet, dass das proximale Schaftende, von lateral quer zum Kopfhals gesehen, etwa in Kopfhalsrichtung verläuft.

## Claims

1. A hip joint prosthesis having a shaft to be inserted in the medullary canal of the femur, which shaft is curved in the anterior/posterior plane and has a centre of curvature situated to the posterior, characterised in that the shaft (1) has in the proximal portion thereof a curvature with the centre of curvature situated to the anterior, whereas the curvature with the posterior situated centre of curvature is provided in the distal portion of the shaft.

2. A prosthesis according to Claim 1, characterised in that the curvature with the anterior situated centre of curvature lies within a distance of approximately 4–8 cm below a socket proximally terminating the shaft.

3. A prosthesis according to Claim 1 or 2, characterised in that the curvature with the anterior situated centre of curvature comprises a change of direction in the anterior/posterior plane of approximately 5–10°.

4. A prosthesis according to one of Claims 1 to 3, characterised in that the proximal shaft end, viewed sideways from the side of the neck of the joint ball, extends approximately in the direction of the ball neck.

## Revendications

1. Prothèse de hanche comportant une tige courbe dans le plan antéro-postérieur, à centre de courbure déplacé postérieurement, destinée à être insérée dans le canal médullaire du fémur, caractérisée en ce que la tige (1) présente, dans la région proximale, une courbure à centre de courbure déplacé antérieurement, tandis que la courbure à centre de courbure déplacé postérieurement se trouve dans la région distale de la tige.

2. Prothèse selon la revendication 1, caractérisé en ce que la courbure à centre de courbure déplacé antérieurement se situe dans les limites d'un segment de 4 à 8 cm environ au-dessous d'une plaquette qui termine la tige à son extrémité proximale.

3. Prothèse selon la revendication 1 ou 2, caractérisée en ce que la courbure à centre de courbure déplacé antérieurement comprend un changement de direction d'environ 5 à 10° dans le plan antéro-postérieur.

4. Prothèse selon l'une quelconque des revendications 1 à 3, caractérisée en ce que l'extrémité proximale de la tige, vue de côté perpendiculairement au col, s'étend approximativement dans la direction du col.

Fig.1          Fig.2